# EUROPÄISCHE PATENTSCHRIFT

(11) **EP 1 437 997 B1**
(45) Veröffentlichungstag und Bekanntmachung des Hinweises auf die Patenterteilung: **02.05.2007**
(21) Anmeldenummer: 02774666.8
(22) Anmeldetag: 30.09.2002
(51) Int. Cl.: A61H 7/00, A45D 34/04, A45D 40/26

(54) **KOSMETISCHES REINIGUNGSPRODUKT**
COSMETIC CLEANING PRODUCT
PRODUIT COSMETIQUE DE NETTOYAGE

(30) Priorität: 04.10.2001 DE 10148933
(43) Veröffentlichungstag der Anmeldung: 21.07.2004
(73) Patentinhaber: Beiersdorf AG, 20245 Hamburg (DE)
(72) Erfinder: LISTE, Kathrin, 22301 Hamburg (DE); ROHDE, Olaf, 25335 Elmshorn (DE); TREU, Jens, 22844 Norderstedt (DE)
(86) Internationale Anmeldenummer: PCT/EP2002/010928
(87) Internationale Veröffentlichungsnummer: WO 2003/030807

(56) Entgegenhaltungen:
- GB-A- 643 633
- US-A- 1 594 636
- US-A- 5 445 596
- PATENT ABSTRACTS OF JAPAN vol. 2000, no. 01, 31. Januar 2000 (2000-01-31) & JP 11 276250 A (IWATA SHOICHI), 12. Oktober 1999 (1999-10-12)

## Beschreibung

Die vorliegende Erfindung betrifft ein kosmetisches Reinigungsprodukt bestehend aus einem Behälter für eine kosmetische Reinigungszubereitung, einer Massagevorrichtung und einer kosmetischen Reinigungszubereitung. Massagevorrichtung und Behälter sind fest miteinander verbunden und werden im weiteren Text als Packmittel bezeichnet. Die feste Verbindung von Massagekopf und Behälter kann auch in der Art und Weise gestaltet sein, das sich die Verbindung Zwecks Nachfüllung lösen lässt. Eine spezielle Anwendung der kosmetischen Reinigungszubereitung ist ein speziell auf die Massagevorrichtung abgestimmtes Duschprodukt.

Behälter - wie beispielsweise Flaschen oder Tiegel dienen u. a. als Aufbewahrungsort von Flüssigkeiten im kosmetischen und dermatologischen Bereich. Die Flaschen sind dabei insbesondere aus einem flexiblem Kunststoff gefertigt, so dass ein leichter Druck auf den Flaschenkörper ausreicht, die in der Flasche befindliche Flüssigkeit aus der Öffnung zu treiben.

Beispielsweise seien hier die bekannten Kunststoffflaschen für Duschzubereitungen, Flüssigseifen, Shampoo angeführt, ohne dass diese Aufzählung vollständig wäre.
Bevorzugt werden Flaschen, die mit einem Schraubdeckel verschlossen werden können.

Die Herstellung der Flaschen beziehungsweise Behälter erfolgt oftmals nach dem Extrusionsblasverfahren.

Aus der US 3 892 829 sind ein Verfahren und eine Vorrichtung zur Herstellung von Flachflaschen aus einem extrudierten Schlauch bekannt, der in einer Zwischenform vorgeblasen und erst dann in eine Endblasform übergeben wird, deren Formnest die Kontur der herzustellenden Flachflasche hat.

Aus der DE 37 02 844 A1 sind ein diesem Prinzip folgendes Verfahren und eine danach arbeitende Extrusionsblasmaschine bekannt. Dabei wird ein Kunststoffschlauch frei extrudiert, in eine Zwischenform übernommen und dort zu einem rotationssymmetrischen Zwischenformling geblasen. Dieser Zwischenformling, der mithin in jeder Höhe einen kreisförmigen Querschnitt hat, hat näherungsweise bereits die Länge (Höhe) der herzustellenden Flachflasche und weist in seinen Hauptabschnitten (Boden, Körper, Hals) einen Umfang auf, der den entsprechenden Umfängen der Flachflasche mehr oder minder angenähert ist. Letztere wird durch Übergabe des Zwischenformlings in eine Endblasform ausgeformt, wie sie beispielsweise aus der DE 27 20 448 C2 bekannt ist.

Diese Technik der weitgehend abfall- und dementsprechend quetschnahtfreien Herstellung von Flachflaschen mit im wesentlichen uniformer Wandstärke hat sich bewährt.

In der EP 0 688 658 A1 wird der Zwischenformling zumindest während der Überführung aus der Zwischenform in die Endblasform von unten (mechanisch) unterstützt.
Der Zwischenformling ist zumindest während der Übergabe aus der Zwischenform in die Endblasform durch ein zusätzliches, bewegliches Formteil abgestützt.
Dieses Formteil kann vorteilhaft der Bodenkontur des Zwischenformlings angepasst sein. In der Regel muß das Formteil in vertikaler Richtung verschiebbar sein, damit das Schließen der Endblasform nicht behindert wird.

Auch Massageapplikatoren sind seit langer Zeit bekannt, beispielsweise offenbart die GB 643 633 A einen Massageapplikator gemäß dem Oberbegriff des Anspruchs 1. Es gibt sie in den verschiedensten Formen und Materialien. Sie können aus Kunststoffen oder natürlichen Materialien bestehen.

Es ist bereits eine Massageeinrichtung bekannt, welche elektrisch ansteuerbar und in einem Sitzmöbel, wie Sessel oder dergleichen, integriert ist. Bei dieser bekannten Massageeinrichtung sind ein oder mehrere Massageköpfe unterhalb des Sitzbezuges angeordnet. Diese Massageköpfe werden elektrisch von Elektromotoren angetrieben, so dass sie in Bewegung versetzt werden und eine auf dem Sitzmöbel sitzende Person an einer entsprechenden Stelle mittels des Massagekopfs massiert werden kann.

Ferner ist eine Massageeinrichtung bekannt, bei welcher sich von einer Kunststoffkugel Massagenoppen über die Kugelfläche verteilt in radialer Richtung erstrecken, so dass diese Kugel zur Massage auf der zu massierenden Fläche bzw. dem menschlichen Körper abgerollt werden kann.

Massageapplikatoren aus Kunststoff können die verschiedensten Formen haben. Es sind Rollen-/Kugelapplikatoren, sowie Noppenapplikatoren bekannt.

Bei den Rollen- oder Kugelapplikatoren werden bewegliche Kugeln in einer geeigneten Führung gehalten und man erreicht auf diese Weise eine reine Druckmassage. Die Druckmassage ist sehr hautfreundlich, da der Reibungswiderstand auf ein Minimum reduziert wird.

Noppenapplikatoren aus Gummi oder weichem Kunststoff zeigen eine erhöhte Reibung. Diese erhöhte Reibung verstärkt den Massageeffekt in den oberen Hautschichten, kann aber auch an empfindlichen Hautpartien zu Hautirritationen führen. Generell wird durch die Massage mit einem Noppenapplikator ein stärkerer Massageeffekt erzielt. Gleichzeitig führt die Massage mit einem Noppenapplikator auch zu höherer mechanischer Belastung der Haut und des darunter liegenden Gewebes und damit zu einer erhöhten Durchblutung der Haut.
Die Erhöhte Durchblutung und die Massage des Unterhaut Gewebes führt zu einer Festigung des Unterhautgewebes. Diese Festigung führt zu einer Verbesserung des Hautbildes und beugt so Cellulite vor oder wirkt ihr entgegen.

Die für Massageapplikatoren aus Kunststoff genannten Punkte gelten generell auch für Massageapplikatoren aus natürlichen Materialien wie zum Beispiel Holz.
Es gibt bei diesen Naturstoffapplikatoren aber auch noch besondere Formen, die beispielsweise aus geflochtenem Sisalseil oder ähnlichen Materialien bestehen. Bei diesen Applikatoren wird die raue Oberfläche der Naturstoffe für die Massage genutzt.

Schon bei einem einfachen Wasserbade ohne Zusatz von Tensiden kommt es zunächst zu einer Quellung der Hornschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab- bzw. ausgewaschen.

Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze noch verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden.

Flüssige Duschzubereitungen in Form von Syndets (synthetischen Detergentien) sind seit langer Zeit bekannt. Es handelt sich dabei im wesentlichen um oberflächenaktive Substanzen oder Stoffgemische, die dem Verbraucher in verschiedenen Zubereitungen angeboten werden. Zubereitungen solcher Art zeichnen sich im allgemeinen durch einen mehr oder weniger hohen Wassergehalt aus, können aber auch beispielsweise als Konzentrat vorliegen. Hierbei handelt es sich heutzutage hauptsächlich um seifenfreie Zubereitungen mit einem pH Wert unter 7. Die Entwicklung solcher Produkte war erst mit der Entdeckung synthetischer Tenside möglich. Seit dieser Zeit sind diese Produkte vielfach weiterentwickelt worden und es gibt ein sehr großes Feld des Standes der Technik. Diese waschaktiven Substanzen haben, wie es dem Fachmann bekannt ist, barriereschädigende Wirkungen. Auch werden durch die verlängerte Applikation nach dem Abspülen mit Wasser mehr Tenside auf der Haut verbleiben. Die Barriereschädigende Wirkung von Tensiden und das verbleiben auf der Haut nach dem Abspülen haben wir bereits in unserer Anmeldung "Verfahren zur Herstellung besonders hautverträglicher kosmetischer oder dermatologischer Reinigungszubereitungen" (DE 199 60 767 A1) ausführlich dargelegt. Insbesondere die intensive Applikation des Duschproduktes mittels Massage führt zu einem erhöhten Irritationsrisiko. Durch die Massage werden die obersten Hautschichten abgelöst und die Hautbarriere geschwächt. Dadurch können die waschaktiven Substanzen in tiefere Hautschichten vordringen und Irritationen hervorrufen.

Bevorzugt handelt es sich bei den fließfähigen Formulierungen um Emulsionen, Suspensionen, Kolloiden, Dispersionen Gelen oder Lösungen.

Es lassen sich auch gelförmige Duschzubereitungen ohne Gelbildner formulieren, da bestimmte Tensidmischungen bei Zugabe von Salzen verdicken.

Im technischen Sinne werden unter Gelen verstanden: Relativ formbeständige, leicht verformbare disperse Systeme aus zumindest zwei Komponenten, welche in der Regel aus einem - meist festen - kolloidal zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrückenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen; Hydrogele, welche praktisch fettfrei sind. Meistens sind Gele durchsichtig. In der kosmetischen beziehungsweise pharmazeutischen Galenik zeichnen sich Gele in aller Regel durch halbfeste, oft fließfähige Konsistenz aus.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele, fachsprachlich auch "surfactant gels" genannt, Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten.

Emulsionen sind metastabile Zwei- oder Mehrphasensysteme, bei welchen die einzelnen Phasen im flüssigen Zustand vorliegen. Die gängigsten Emulsionen sind O/W- und W/O-Emulsionen. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca. 1 µm bis ca. 50 µm. Solche "Makroemulsionen" sind, ohne weitere färbende Zusätze, milchigweiß gefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulichweiß gefärbt und undurchsichtig.

Mizellaren und molekularen Lösungen mit Partikeldurchmessern kleiner als ca. 10⁻² µm erscheinen klar und transparent.

Der Tröpfchendurchmesser von transparenten beziehungsweise transluzenten Mikroemulsionen dagegen liegt im Bereich von etwa 10⁻² µm bis etwa 10⁻¹ µm. Solche Mikroemulsionen sind meist niedrigviskos. Die Viskosität vieler Mikroemulsionen vom O/W-Typ ist vergleichbar mit der des Wassers.

Den bei weitem wichtigsten Produkttyp im Bereich der Hautpflegemittel beziehungsweise im Bereich kosmetischer und/oder dermatologischer Zubereitungen stellen Emulsionen dar. Emulsionen sind disperse Zwei- oder Mehrphasensysteme, wobei kosmetische Emulsionen aus mindestens einer Fettphase (Fette und mineralische Öle, Fettsäureester, Fettalkohole etc.) und mindestens einer Wasserphase (Wasser, Glycerin, Glykole usw.) bestehen, die mit Hilfe von Emulgatoren in Form feinster Tröpfchen ineinander verteilt werden. Sind die beiden Flüssigkeiten Wasser und Öl und liegen Öltröpfchen fein verteilt in Wasser vor, so handelt es sich um eine Öl-in-Wasser-Emulsion (O/W-Emulsion, zum Beispiel Milch). Der Grundcharakter einer O/W-Emulsion ist durch das Wasser geprägt. Bei einer Wasser-in-Öl-Emulsion (W/O-Emulsion, zum Beispiel Butter) handelt es sich um das umgekehrte Prinzip, wobei der Grundcharakter hier durch das Öl bestimmt wird.

Die Ölphase wird vorteilhaft gewählt aus der Gruppe der Ester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren und gesättigten und/oder ungesättigten, verzweigten und/oder ungesättigten Alkoholen, aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride. Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft kann die Ölphase einen Gehalt an cyclischen oder linearen Silikonölen, beispielsweise Cyclomethicon (Octamethylcyclotetrasiloxan), aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Die hier und im folgenden beschriebenen Emulsionen lassen sich also entsprechend unter teilweiser oder vollständiger Verwendung von Silikonölen als Silikonemulsionen fertigen. Entsprechendes gilt für die übrigen ölhaltigen Zubereitungen.

Dem Fachmann ist eine Vielzahl von Möglichkeiten bekannt, stabile O/W-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milch, die in diesem Temperaturbereich eher fließfähig sind und sich besonders günstig mit dem erfindungsgemäßen Behälter aufbewahren lassen.

Die Stabilität von Emulsionen ist unter anderem von ihrer Viskosität, insbesondere von der Viskosität der äußeren Phase abhängig. Eine Emulsion wird dann instabil, wenn sich die feindispergierten Teilchen wieder zu größeren Aggregaten zusammenballen und die sich berührenden Tröpfchen zusammenfließen. Dieser Vorgang wird als Koaleszenz bezeichnet. Der Prozess der Koaleszenz läuft desto langsamer ab, je viskoser die äußere Phase der Emulsion ist.

O/W-Emulsionen werden dementsprechend in der Regel durch Verdickungsmittel, welche die Viskosität der wässrigen Phase erhöhen, stabilisiert. Hierzu eignen sich beispielsweise Polyacrylate (Carbomer) und weitere organische Verdickungsmittel. Ein Nachteil dieser Methode der Stabilitätsverbesserung ist die Empfindlichkeit dieser Formulierungen gegen Elektrolyte. Ferner sind auf diese Weise naturgemäß vornehmlich höherviskose Formulierungen (wie Cremes oder Salben) herzustellen.

Emulsionen von "flüssiger" (= fließfähiger) Konsistenz finden in der Kosmetik beispielsweise als Pflege-, Reinigungs-, Gesichts- oder Handlotion Verwendung. Sie haben in der Regel eine Viskosität von etwa 2000 mPa·s bis zu etwa 10 000 mPa·s. Der Stabilität von fließfähigen Emulsionen ist besondere Aufmerksamkeit zu widmen, da die erheblich größere Beweglichkeit der Teilchen eine schnellere Koaleszenz fördert.

Herkömmliche Emulgatoren können entsprechend ihrem hydrophilen Molekülteil in ionische (anionische, kationische und amphotere) und nichtionische untergliedert werden:
Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
Wichtige Vertreter der kationischen Emulgatoren sind die quartären Ammonium-Verbindungen.
Der hydrophile Molekülteil nichtionischer Emulgatoren besteht häufig aus Glycerin, Polyglycerin, Sorbitanen, Kohlenhydraten beziehungsweise Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.
Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man zum Beispiel Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann zum Beispiel erreicht werden, wenn ausgenutzt wird, dass feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei nicht die chemischen, sondern die Oberflächeneigenschaften der Feststoffpartikel.

Eine relativ neue technische Entwicklung ist es, kosmetische oder dermatologische Zubereitungen nur durch feinstverteilte Feststoffteilchen zu stabilisieren. Solche "emulgatorfreien" Emulsionen werden nach ihrem Erfinder auch als Pickering-Emulsionen bezeichnet. Eine Möglichkeit, eine Feststoffstabilisierung in einer kosmetischen oder dermatologischen Zubereitung vorzunehmen, ist nach May-Alert (Pharmazie in unserer Zeit, 15. Jahrg. 1986, Nr. 1, 1-7) beispielsweise, Emulgatorgemische zu verwenden, die sowohl anionische als auch kationische Tenside enthalten. Da beim Zusammengeben von Anion- und Kationtensiden immer unlösliche, elektroneutrale Verbindungen ausfallen, lässt sich durch gezieltes Ausfällen dieser neutralen Tenside in der Grenzfläche ÖI/Wasser eine zusätzliche Feststoffstabilisierung im Sinne einer Pickering-Emulsion erreichen.

Ferner beschreibt die WO 98/42301 A1 emulgatorfreie feindisperse Systeme vom Typ Wasser-in-ÖI, welche durch den Zusatz mikronisierter, anorganischer Pigmente stabilisiert werden, die aus der Gruppe der Metalloxide, insbesondere Titandioxid gewählt werden.

Emulgatorfreie Präparate auf Basis sogenannter Hydrodispersionen sind seit einiger Zeit für den Verbraucher zugängig. Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen wie Emulsionen metastabile Systeme dar und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wässrigen Phase kann die Stabilität eines solchen Systems beispielsweise dadurch gewährleistet werden, dass in der wässrigen Phase ein Gelgerüst aufgebaut wird, in welchem die Lipidtröpfchen stabil suspendiert sind.

W/O-Lipodispersionen sind in umgekehrter Analogie emulgatorfreie feindisperse Zubereitungen vom Typ Wasser-in-Öl.

Bei der Verwendung eines Standard Duschproduktes mit einem der oben genannten Applikatoren kam es in der Vergangenheit zu folgenden negativen Produkteigenschaften:
- Duschprodukt und Massageapplikator waren nicht aufeinander abgestimmt, man erzielte einen zu starken oder zu schwachen Massageeffekt.
- Die Intensität der Massage war nicht variabel und ließ sich somit nicht auf verschiedene Hautzonen einstellen.
- Die lange Einwirkzeit des Duschproduktes während der Massage führte zu Hauirritationen.
- Applikator war nicht auf das Duschprodukt abgestimmt. Eventuell, ließ sich der Applikator nur schlecht oder gar nicht reinigen oder die Oberfläche wurde durch das Duschprodukt angegriffen oder zersetzt.
- Applikator und Duschprodukt waren nicht aufeinander abgestimmt, so dass es bei unsachgemäßer Handhabung zu Verkeimungen des Duschproduktes und Applikators kommen konnte.

Bisher musste man bei einer Massage mittels Applikator unter der Dusche immer auf ein Standard Duschprodukt und einen der oben genannten Applikatoren zurückgreifen, spezielle Massageduschprodukte sind bis jetzt noch nicht bekannt.

Aufgabe der Erfindung ist es, einen Behälter zur Verfügung zu stellen, welcher eine optimale Verbindung zwischen einem Aufbewahrungsort und einer Massageeinrichtung bietet, so dass eine Auftrennung der beiden Funktionen in zwei getrennte Einrichtungen nicht mehr erforderlich ist. Außerdem mussten speziell für diese Anwendung geeignete Duschproduktformulierungen gefunden werden.

Gelöst wird diese Aufgabe überraschend durch ein kosmetisches Reinigungsprodukt wie es im Hauptanspruch beschrieben wird. Die Unteransprüche betreffen vorteilhafte Ausführungsformen dieses Reinigungsproduktes, insbesondere seines Massageapplikators und der enthaltenden kosmetischen Reinigungszubereitungen. Beansprucht wird weiterhin die Verwendung eines solchen Reinigungsproduktes als Duschzubereitung.

Mit dem erfindungsgemäßen Behälter können flüssige und fließfähige Stoffe sowie leicht verteilbare feste Stoffe sowie Mischungen aus zwei oder mehreren Komponenten aufbewahrt werden, wobei die Massageeinrichtung gleichzeitig das Einmassieren der abgegebenen Stoffe in die Haut erleichtert.

Der Behälter ist hervorragend geeignet für Emulsionen, Suspensionen, Dispersionen, Lösungen (gasförmiger, flüssiger und fester Stoffe), Kolloide und dergleichen, sehr bevorzugt zum Auftragen von kosmetischen oder dermatologischen Mitteln auf die Haut, insbesondere von Gelen, Emulsionen, Pickering-Emulsionen, Hydrodispersionen, Lipodispersionen.

Gemäß einem besonderen Aspekt liegt der Erfindung die Aufgabe zugrunde, eine flexibel verwendbare und kostengünstig herstellbare Massagevorrichtung zu schaffen.

Gemäß einem besonderen Aspekt liegt der Erfindung die Aufgabe zugrunde, eine Massagevorrichtung zu schaffen, welche einen individuell angepassten Massageeffekt bewirkt.

Im folgenden wird das Packmittel anhand der Figuren näher erläutert, wobei hierdurch die Erfindung nicht beschränkt werden soll.

Erfindungsgemäß, wird insbesondere eine Massagevorrichtung vorgeschlagen, welche eine erste Oberfläche aufweist, die beim Massieren die zu massierende Fläche kontaktiert, wobei ein vorbestimmter Massagekennwert mittels einer Einstelleinrichtung einstellbar ist.

Die erfindungsgemäße Massagevorrichtung ist insbesondere derart gestaltet, dass sie eine Massagenoppeneinrichtung mit wenigstens einer Noppe aufweist sowie mit einer ersten Oberfläche, die beim Massieren die zu massierende Fläche kontaktiert.

Eine Massagenoppeneinrichtung ist im Sinne der vorliegenden Erfindung insbesondere eine Einrichtung, welche eine oder mehrere Noppen aufweist, an denen die erste Oberfläche angeordnet ist.
Der Begriff "Noppe" ist im Sinne der vorliegenden Erfindung weit gefasst zu verstehen und erstreckt sich insbesondere auf Profilierungserhöhungen einer Außenkontur dieser Massagenoppeneinrichtung. Gegebenenfalls ist die Massagenoppeneinrichtung bzw. die Noppe auch als im wesentlichen unprofilierte Oberfläche bzw. als nicht erhöhte Oberfläche gestaltet. Besonders bevorzugt sind jedoch mehrere Noppen vorgesehen, welche sich mit zylindrischer Außenkontur oder kugelsegmentförmiger oder halbkugelförmiger oder dreieckiger oder pyramidenförmiger oder sonstiger Außenkontur von einer Oberfläche der Massagenoppeneinrichtung bzw. eines Grundkörpers erstrecken.

Die zu massierende Fläche ist im Sinne der vorliegenden Erfindung insbesondere eine Außenoberfläche des menschlichen Körpers, wie die Haut des menschlichen Körpers.

Während der Massage wird von der Massagevorrichtung bzw. der Massagenoppeneinrichtung bzw. der ersten Oberfläche eine Kraft auf die zu massierende Fläche übertragen. Diese Kraft bewirkt insbesondere, dass die Massage der zu massierenden Fläche sensitiv wahrnehmbar ist.

Im Bereich der ersten Oberfläche ist eine vorbestimmte Härte und/oder eine vorbestimmte Elastizität und/oder eine vorbestimmte Geometrie dieser Oberfläche gegeben. Diese Härte, Elastizität sowie Geometrie ist insbesondere durch die Geometrie und/oder das Material des diese erste Oberfläche tragenden und/oder der an das diese erste Oberfläche tragenden Bauteil angrenzenden Bauteile und/oder die jeweilige Relativanordnung zueinander bestimmt.

Ein Massagekennwert ist im Sinne der vorliegenden Erfindung insbesondere ein Kennwert, welcher die Kraft- und/oder Energieübertragung zwischen der ersten Oberfläche und der zu massierenden Fläche zumindest mitbestimmt, und zwar insbesondere hinsichtlich der flächenmäßigen und/oder zeitlichen Verteilung dieser übertragenden Kraft oder Energie. Vorzugsweise ist ein Massagekennwert im Sinne der vorliegenden Erfindung die Härte oder die Elastizität und/oder die Geometrie im Bereich der ersten Oberfläche.

Die Formulierung "im Bereich der ersten Oberfläche" ist im Sinne der vorliegenden Erfindung insbesondere derart zu verstehen, dass der tatsächlich gegebene Kennwert der ersten Oberfläche bzw. des diese erste Oberfläche tragenden Bauteils, wie Elastizität oder härte oder dergleichen, gegeben ist, oder dahingehend, dass der entsprechende (Ersatz)Kennwert im Zusammenwirken mit anderen Bauteilen an dieser ersten Oberfläche bewirkt wird.

Im folgenden wird ein derartiges Zusammenwirken beispielhaft erläutert, ohne dass die Erfindung hierdurch beschränkt werden soll:

Eine oder mehrere Noppen sind jeweils hohlzylindrisch gestaltet und weisen in einem ersten Stirnbereich, gegebenenfalls eine abgerundete oder halbkreisförmige stirnseitige Begrenzungswandung auf, und wobei in dem zweiten, dem ersten gegenüberliegenden Stirnbereich, eine Öffnung zur Aufnahme eines, beispielsweise kolbenförmigen, Doms vorgesehen ist, welcher axial innerhalb der Noppe verschieblich ist. Die Noppe ist im wesentlichen dünnwandig gestaltet und besteht aus einem im wesentlichen flexiblen Material. Dieses Material weist eine vorbestimmte Elastizität sowie eine vorbestimmte Härte auf. wenn die Massagevorrichtung zur Massage belastet wird und ein vorbestimmter Außenoberflächenbereich der Noppe die zu massierende Fläche kontaktiert, ist, insbesondere bedingt durch das Material und die geometrische Gestaltung der Noppe, die Wirkung einer vorbestimmten Elastizität dieser Noppe bei der Belastung der zu massierenden Fläche sowie eine bestimmte Härtewirkung gegeben. Diese im Bereich der ersten Oberfläche gegebene Härtewirkung sowie Elastizität kann dadurch verändert werden, dass der kolbenförmige Innenkörper innerhalb der Noppe axial verschoben wird, wodurch im Bereich der ersten Oberfläche erfassbare Kennwerte, wie die Härte oder die Elastizität oder die Knickfestigkeit der Noppe oder dergleichen verändert werden. Diese Wirkung in einer beispielhaften Ausführungsform dadurch erzeugt, dass die im Bereich der ersten Oberfläche messbare Steifigkeit der Gesamteinrichtung aus Noppen und darin angeordneten Kolben verändert wird.

Diese vorstehende beispielhafte Gestaltung sowie das Zusammenwirken der vorerwähnten unterschiedlichen Elemente der erfindungsgemäßen Vorrichtung soll die Erfindung nicht beschränken. Bevorzugt ist, dass durch das Zusammenwirken unterschiedlicher Bauelemente die Geometrie der ersten Oberfläche oder einer an diese erste Oberfläche angrenzenden Oberfläche verändert werden kann.

Besonders bevorzugt ist ein Bauteil mit der ersten Oberfläche vorgesehen, welches verhältnismäßig weich und/oder elastisch im Vergleich zu einem oder mehreren weiteren Bauteilen gestaltet ist, welche gegenüber dem die erste Oberfläche tragenden Bauteil relativ beweglich angeordnet sind und bei einer Relativbewegung die Geometrie der ersten Oberfläche oder einer an diese erste Oberfläche angrenzenden Oberfläche verändern.

Vorzugsweise ist der Massagekennwert der Massagenoppeneinrichtung, wie Härte und/oder Elastizität oder Geometrie im Bereich der ersten Oberfläche der Massagenoppeneinrichtung, stufenlos einstellbar. Bevorzugt weist die Massagevorrichtung bzw. die Massagenoppeneinrichtung bzw. die Einstelleinrichtung wenigstens ein erstes Element sowie wenigstens ein zweites Element auf, welche relativbeweglich zueinander angeordnet sind. In unterschiedlichen Relativstellungen dieser Elemente sind unterschiedliche Massagekennwerte, wie Härte und/oder Elastizität und/oder Geometrie, im Bereich der ersten Oberfläche der Massagevorrichtung gegeben.

Vorzugsweise ist das zweite Element wenigstens teilweise im ersten Element oder in einer Vertiefung des ersten Elements angeordnet oder anordbar. Besonders bevorzugt ist das erste Element ein auswechselbarer oder verstellbarer Aufsatz, welcher auf wenigstens ein zweites Element aufgesetzt werden kann oder aufgesetzt ist.

In einer besonders bevorzugten Ausführungsform der Erfindung ist wenigstens ein zweites Element ein auswechselbarer oder verstellbarer Einsatz, welcher in wenigstens ein zweites Element eingesetzt werden kann oder welcher innerhalb eines wenigstens zweiten Elements angeordnet ist.

Gemäß einer besonders bevorzugten Ausführungsform der Erfindung ist wenigstens ein erstes Element zumindest teilweise hohl gestaltet.

Wenigstens ein zweites Element weist vorzugsweise einen außenliegenden Außenoberflächenbereich auf, welcher unter vorbestimmten Gegebenheiten einen innenliegenden Innenoberflächenbereich wenigstens eines zweiten Elementes kontaktiert. Dieser Kontakt kann insbesondere bei vorbestimmten oder bei allen Relativstellungen zwischen dem ersten Element und dem zweiten Element gegeben sein, wobei der Flächeninhalt der kontaktierenden Fläche konstant oder veränderlich ist.

Bevorzugt ist wenigstens ein erstes Element axial beweglich innerhalb eines zweiten Elements angeordnet. Diese axiale Beweglichkeit ist insbesondere derart gestaltet, dass das zweite Element längsverschieblich innerhalb des ersten Elements angeordnet ist.

Besonders bevorzugt ist die Außenkontur wenigstens eines zweiten Elements mit einem Außengewinde versehen und die Innenkontur wenigstens eines ersten Elements mit einem Innengewinde, so dass das Außengewinde des zweiten Elements in das Innengewinde des ersten Elements eingreifen kann.

Erfindungsgemäß ist insbesondere vorgesehen, dass die erste Oberfläche der Massagevorrichtung, welche bei der Massage die zu massierende Fläche kontaktiert, eine Oberfläche, wie Außenoberfläche, des ersten Elements ist. Wenigstens ein erstes Element ist, bevorzugt im Bereich seiner Außenoberfläche, und zwar insbesondere im Bereich der ersten Oberfläche der Massagenoppeneinrichtung bzw. der Massagevorrichtung, weicher gestaltet, als das zweite Element in einem vorbestimmten Außenoberflächenbereich dieses zweiten Elements. Dieser vorbestimmte Außenoberflächenbereich ist besonders bevorzugt ein Oberflächenbereich, welcher sich unter vorbestimmten Gegebenheiten gegen eine Innenoberfläche des ersten Elements abstützt, und zwar gegebenenfalls bei einer vorbestimmten Relativstellung zwischen dem ersten Element und dem zweiten Element in einem Bereich der Innenoberfläche des ersten Elements, welcher dem als weicher bezeichneten Außenoberflächenbereich dieses Elements zugeordnet ist.

Wenigstens ein erstes Element unterscheidet sich vorzugsweise von einem zweiten Element durch sein Material, wobei besonders bevorzugt das erste Element aus einem weicheren Material besteht als das zweite Element.

Das erste Element sowie das zweite Element ist jeweils aus einem oder aus verschiedenen Materialien gefertigt.

Bevorzugt ist das erste und/oder das zweite Element aus Kunststoff, wie Polyethylen oder Polypropylen oder dergleichen, gefertigt. Besonders bevorzugt ist das erste und/oder das zweite Element aus einem Thermoplast und/oder Duroplast und/oder Elastomer gefertigt.

Vorzugsweise weist das Material, aus welchem das erste Element gefertigt ist, wenigstens einen Werkstoffkennwert auf, welcher sich von einem Werkstoffkennwert des zweiten Elements unterscheidet.

Bevorzugt ist wenigstens ein Oberflächenbereich der ersten Oberfläche der Massagenoppeneinrichtung im wesentlichen abgerundet gestaltet.

Vorzugsweise ist wenigstens ein erstes Element eine im wesentlichen hohl gestaltete Noppe und wenigstens ein zweites Element dornartig gestaltet, wobei sich dieses zweite Element in den Hohlraum dieser Noppe erstreckt oder erstrecken kann. Die dornartige Gestaltung dieses zweiten Elements soll die Erfindung nicht hinsichtlich der Gestaltung der Oberflächenkontur des zweiten Elements beschränken.

Insbesondere ist das zweite Element in der Richtung, in welcher es sich in die Noppe erstreckt, länger gestaltet, als in seinen Abmaßen der senkrecht zu dieser Richtung angeordneten Querschnittsfläche. Bevorzugt ist ferner, dass das zweite Element in der vorher erwähnten Richtung kürzer gestaltet ist als die Abmaße der benannten Querschnittsfläche.

Besonders bevorzugt ist das zweite Element in das erste Element einführbar bzw. im ersten Element verstellbar, wobei bei unterschiedlicher Einführtiefe bzw. bei vorbestimmten unterschiedlichen Relativpositionen zwischen dem ersten Element und dem zweiten Element unterschiedliche Massagekennwerte wie (Ersatz)Härte und/oder (Ersatz)Elastizität oder dergleichen im Bereich der ersten Oberfläche gegeben sind.

Eine bevorzugte erfindungsgemäße Massagevorrichtung weist eine Arretiereinrichtung auf, mittels welcher wenigstens ein erstes Element gegenüber wenigstens einem zweiten Element in einer vorbestimmten Relativposition gehalten und/oder arretiert werden kann.

Besonders bevorzugt ist wenigstens ein zweites Element gegenüber wenigstens einem ersten Element bzw. innerhalb wenigstens eines ersten Elements in wenigstens einer vorbestimmten Relativstellung zwischen diesen Elementen klemmbar.

Besonders bevorzugt ist die Massagevorrichtung manuell betätigbar. Vorzugsweise ist die Massagevorrichtung mechanisch betätigbar. Besonders bevorzugt ist die Massagevorrichtung nicht-elektrisch betätigbar.

Vorzugsweise ist wenigstens ein Massagekennwert unabhängig von der Größe und/oder dem zeitlichen Verlauf der Energie- und/oder der Kraft einstellbar, welche bei der Massage in die Massage Vorrichtung eingeleitet wird. Die Massagewirkung kann mittels der erfindungsgemäßen Massagevorrichtung insbesondere derart eingestellt werden, dass eine unterschiedliche Massagewirkung gegeben ist bzw. eine unterschiedliche Massagewirkung sensitiv spürbar ist, wenn die Massagevorrichtung, gegebenenfalls manuell, mit einer vorbestimmten Energie und/oder Kraft versorgt wird und die Einstelleinrichtung in unterschiedlichen Einstellstellungen geschaltet ist.

Ein erfindungsgemäßes Packmittel ist so gestaltet, das die erfindungsgemäße Massagevorrichtung als Behälterdeckel angeordnet ist.

Besonders bevorzugt ist erfindungsgemäß vorgesehen, dass eine erfindungsgemäße Massagevorrichtung an einem Behältnis vorgesehen ist, welches als Duschgelflasche oder Shampooflasche oder als Seifenbehälter gestaltet ist, oder an einer sonstigen Flasche bzw. einem sonstigen Behältnis zur Aufnahme von Fluiden oder an einem Behälter zur Aufnahme von festen oder gasförmigen Stoffen.

Ein besonderer Vorteil dieser Verpackung ist, die leicht durchzuführende Reinigung der Massagekörper, da durch die spezielle Konstruktion ein ungehinderter Wasserdurchtritt durch die Führungskörper gewährleistet ist. Dadurch kann auf eine hohe Konservierungsmittelkonzentration im Reinigungsmittel verzichtet werden. Die mikrobiologische Sicherheit des Produkts ist auch so gewährleistet.

Die spezielle Konstruktion der Massageköper ermöglich die reine Druckmassage, so werden Hautreizungen durch übermäßige Reibung auf der Hautoberfläche verhindert. Auf diese Weise kommt es zu einer sehr schonenden Anwendung der Reinigungsmittel. Die Haut wird durch die Anwendung von einem Reinigungsmittel während einer Massage weniger gereizt

Im Sinne der Erfindung wird vorzugsweise ein runder, abgewinkelter Massagekopf verwendet und dadurch die Massage erheblich vereinfacht. Der Massagekopf ist vorzugsweise oval und in spezieller, konzentrischer Anordnung ausgelegt.

Als erfindungsgemäßes Ventilsystem eignen sich Ventilsysteme zum dosierten Auslassen einer Substanz aus einem Behälter, wobei für das Auslassen der Substanz aus dem Behälter eine Abgabeöffnung durch zumindest eine der Wände des Behälters und für eine Rückbelüftung eine Belüftungsöffnung durch zumindest eine der Wände des Behälters vorgesehen ist; mit einem ersten Ventil, welches die Abgabeöffnung für die Substanz abdichtet, solange eine Druckdifferenz zwischen dem Behälterinneren und dem Behälteräußeren kleiner als ein erster vorgegebener Grenzwert ist, und welches die Abgabeöffnung für die Substanz öffnet, wenn die Druckdifferenz zwischen dem Behälterinneren und dem Behälteräußeren größer als der erste vorgegebene Grenzwert ist, sowie mit einem zweiten Ventil, welches die Belüftungsöffnung für die Rückbelüftung öffnet, solange eine Druckdifferenz zwischen dem Behälterinneren und dem Behälteräußeren kleiner als ein zweiter vorgegebene Grenzwert ist, und welches die Belüftungsöffnung für die Rückbelüftung abdichtet, wenn die Druckdifferenz zwischen dem Behälterinneren und dem Behälteräußeren größer als der zweite vorgegebene Grenzwert ist, dadurch gekennzeichnet, dass
das zweite Ventil aus einem flexiblen Material besteht und in Form einer Lippe ausgebildet ist, welche in der Lage ist, die Belüftungsöffnung zu verschließen.

Erfindungsgemäß befindet sich die Rückbelüftungsöffnung in dem ersten Ventil und/oder das zweite Ventil in das erste Ventil integriert ist.
Erfindungsgemäß ist zumindest eines der Ventile aus einem flexiblen Material besteht und in Form einer Lippe ausgebildet ist, welche in der Lage ist, sich zur Abdichtung über eine Öffnung zu legen.

Der erfinderische Behälter soll im folgenden durch einige Abbildungen veranschaulicht werden, ohne sich durch die Wahl der abgebildeten Beispiele unnötig beschränken zu wollen.
Es zeigen:

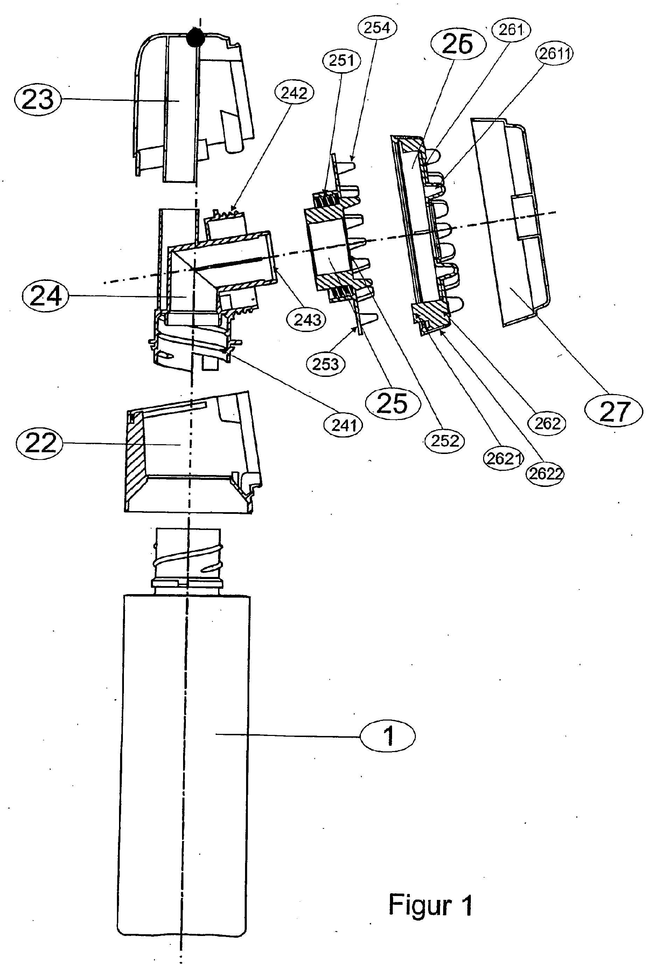

- Figur 1: Explosionszeichnung einer besonders vorteilhaften Ausführung der erfindungsgemäßen Massageapplikators, mit besonders ausgeführten Behälter in Flaschenform und mehreren zur Vorrichtungen gehörenden Einzelteilen (Flasche (1), Massagevorrichtung (2) bestehend aus: (22) untere Kopfkappe, (23) obere Kopfkappe, (24) Winkelstück mit Flaschengewinde (241), Massagegewinde (242) und Ventilsitz (243), (25) Massagenoppeninnenteil mit Massagehärteverstellgewinde (251) und Ventilausschnitt (252), (26) Massagenoppenaussenteil bestehend aus Massagenoppenscheibe (261) aus weichem Material und Siebplatte mit Drehring (262) aus hartem Material, (27) Abdeckkappe) in der seitlichen Ansicht,

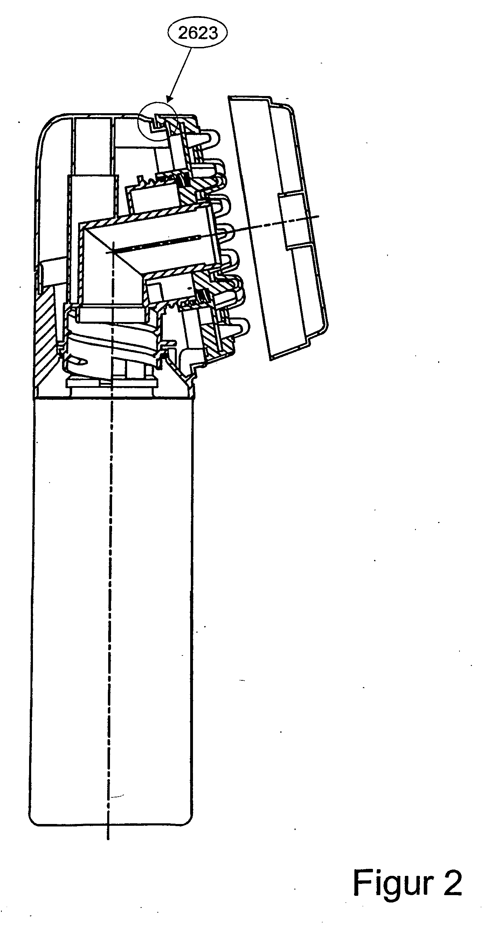

- Figur 2: Schnitt durch eine besonders vorteilhafte Ausführung des erfindungsgemäßen, aus den in Figur 1 dargestellten Einzelteilen zusammengesetzten, Massageapplikators, mit einem besonders vorteilhaft ausgeführten Behälter in Flaschenform und aufgesetzter Massagevorrichtung, die aus mehreren Vorrichtungen gebildet wird, in der seitlichen Ansicht

Figur 1 zeigt eine spezielle Ausführung des erfindungsgemäßen Massagebehälters. Er besteht im wesentlichen aus sieben Teilen, davon bilden sechs Teile die Massagenoppeneinrichtung (2) als Explosionszeichnung.

Die Flasche (1), die als Produktbehälter dient, ist von einer zweiteiligen Kappe -Teile (22) und (23) - mit einem innenliegenden Winkelstück (24) verschlossen.
Die Flasche (1), nach dem Extrusionsblasverfahren hergestellt, ist im wesentlichen von rechteckiger Gestalt, wobei die Kanten der Flasche (1) abgerundet sind.
Die Flasche weist an ihrem Ende des Flaschenhalses (11) ein Gewinde (12) auf, das eine feste Schraubverbindung zum Gewinde (241) des Winkelstücks einzugehen vermag. Die zweite Seite des Winkelstücks weist ebenfalls einen Gewindeansatz (242) auf, auf den der verstellbare Teil der Massagenoppeneinrichtung - das Massagenoppeninnenteil (25) - aufgeschraubt wird. Auf diese Anordnung wird das Massagenoppenaussenteil (26) aufgesteckt, wobei die Mitnehmer (621) der Massagenoppensiebplatte (262) in die Aussparungen (253) des Massagenoppeninnenteils (25) einrasten. Gleichzeitig wird im Bereich des Drehringes (2622) eine Gleitverbindung mit der unteren und oberen Kopfkappe im Bereich (2623 im Figur 2) geschaffen. Ein Drehen am Ring führt zu einer Bewegung des Massagenoppeninnenteils perpendikular zur Drehrichtung und damit zum hinein bzw. herausfahren der starren Noppen (254) in bzw. aus den hohlen Massagenoppen (2611), was eine Änderung der Massagewirkung zu Folge hat.
Der Benutzer des Inhalt der Flasche (1) kann durch leichten Druck auf die Flasche (1) einen Teil des Inhalts durch das im Bereich (243) sitzende Ventil entnehmen und auf seiner Haut beispielsweise auftragen. Durch die Massageeinrichtung kann er nun den aufgetragenen Inhalt bequem in die Haut einmassieren, ohne dass er ein zusätzliches Gerät greifen oder suchen müsste.

In der Figur 2 ist der Massageapplikator aus Figur 1 zum besseren Verständnis in seiner zusammengesetzten Form dargestellt.

Die Erfindung soll durch die beispielhaften und bevorzugten Ausführungsformen nicht beschränkt werden.

Teil der Erfindung ist auch die Verwendung des zuvor beschriebenen Packmittels in Verbindung mit kosmetischen oder dermatologischen Zubereitungen in Form von Gelen, Emulsionen, Mikroemulsionen, Suspensionen, Dispersionen, Kolloiden, Pudern, Pulvern und/oder Pasten vorliegen.

Besonders vorteilhaft ist die Kombination des erfindungsgemäßen Behälters mit flüssigen kosmetischen Reinigungsmitteln, die durch den speziellen Behälter zur massierenden Reinigung verwendet werden können.

Unter die flüssigen kosmetischen Reinigungsmittel fallen alle Formulierungen mit anionischen, kationischen, nichtionischen und amphoteren, beziehungsweise zwitterionischen Tensiden.

Erfindungsgemäß können in diesen Formulierungen Hautpflegesubstanzen enthalten sein. Als Hautpflegesubstanzen können Rückfetter, Conditioner, Peelingkörper oder Wirkstoffe zum Einsatz kommen.

Durch die Verwendung eines speziellen Duschproduktes mit abgestimmten Applikator sind alle oben genannten Punkte entscheidend verbessert worden.

Durch die Verwendung einer Duschgelflasche mit integriertem Massageaufsatz ist gewährleistet, dass Produkt und Massageapplikator gut aufeinander abgestimmt sind und eine unsachgemäße Anwendung durch den Verbraucher somit ausgeschlossen ist.
Massageapplikator und Duschprodukt sind so aufeinander abgestimmt, dass ein optimaler Massageeffekt erzielt wird.

Die Viskosität der Duschformulierung ist gezielt auf diese Anwendung abgestimmt und auch die Gleitfähigkeit der Formulierung auf der Haut ist optimiert. Somit erzielt der Verbraucher einen optimalen, hautschonenden Massageeffekt.

Dadurch, dass Massageapplikator und Duschproduktbehälter fest bzw. nicht ohne weiteres trennbar miteinander verbunden sind, wird die Gefahr der Verkeimung minimiert.

Gleichzeitig ist durch eine entsprechend technische Ausführung der Flasche und oder des Applikators (z. B. mit einem Ventil) sichergestellt, dass keine Fremdstoffe, wie zum Beispiel Wasser oder Hautpartikel, in das Packmittel gelangen können. Somit wird einer gefährlichen Verkeimung des Füllgutes vorgebeugt. Auf diese Art und Weise kann auf eine hohe Konzentration von Konservierungstoffen im Füllgut verzichtet werden, was der Verträglichkeit des Produktes zu Gute kommt.

Konservierungsmittel sind unter Dermatologen für ihre allergieauslösende- und hautschädigende Wirkung bekannt. Durch einen Verzicht auf Konservierungsmittel kann die Verträglichkeit dieses Produktes erheblich verbessert werden.
Dies ist besonders bei dieser Applikationsform - Dusche mit Massage - wichtig, da das Produkt durch die Massage erheblich länger auf der Haut verbleibt. Die längere Applikationsdauer begünstigt das Auftreten von Irritationen durch Konservierungsmittel und waschaktive Substanzen.

Neben den bereits geschilderten Vorteilen besitzen die erfindungsgemäßen Behälter im Bereich der Körperpflege aufgrund des durch die veränderte Oberfläche hervorgerufenen Massageeffektes einen zusätzlichen Vorteil für den Anwender. Während des Auftragens von kosmetischen oder dermatologischen Zubereitungen kann gleichzeitig ein positiver Effekt beispielsweise für die Hautstraffung oder gegen Cellulitis erzielt werden.

Die Duschformulierung ist dermaßen gewählt, das eine Reinigung des Applikators nach Gebrauch problemlos möglich ist.

Gleichzeitig verbessert das Ventil im Applikator entscheidend die Dosierung des Duschproduktes während der Anwendung (Massage).

Ist der Applikator in der Art und Weise gestaltet, dass die Massageintensität variabel ist, dann ist die Massage selbst empfindlicher Körperpartien möglich.

Die folgenden Beispiele, in welchen Waschpräparate zur Haar- und Körperreinigung beschrieben werden, für die der erfinderische Behälter hervorragend Anwendung finden kann, sollen die erfindungsgemäßen Zusammensetzungen erläutern, ohne dass aber beabsichtigt ist, die Erfindung auf diese Beispiele zu beschränken. die Zahlenwerte in den Beispielen bedeuten Gewichtsprozente, bezogen auf das Gesamtgewicht der jeweiligen Zubereitung:

### Beispiel 1:

| | |
|---|---|
| Natrium Laurethsulfat | 11% |
| Cocoamidopropylbetain | 2,5% |
| Natriumcocoylglutamat | 1,5% |
| PEG-40 hydriertes Rizinusöl | 0,5% |
| PEG-100 hydriertes Glycerylpalmitat | 0,5% |
| Natriumbenzoat | 0,45% |
| Natriumsalicylat | 0,2% |
| Citronensäure | 0,5% |
| Parfum | q.s. |
| Wasser | ad 100 |

Zusätzlich noch folgende weitere Rohstoffe: Perlglanz Wachse (1% bis 3%), Trübungsmittel (Wachse oder Polymere) (0,5% bis 3%), Polyquaternium-10 (0,1% bis 0,3%), Trisodium EDTA (0,2% bis 0,6%)

Diese Zubereitung zeichnet sich durch eine extrem verminderte Adsorption von Laurylethersulfat an die Haut aus, das für seine hautirritierende Wirkung bekannt ist. Daher eignet sich diese Rezeptur sehr gut für die erfindungsgemäße Applikationsform.

### Beispiel 2:

| | |
|---|---|
| Natrium Myrethsulfat | 4% |
| Natrium Laurethsulfat | 3% |
| Cocoamidopropylbetain | 2% |
| Natriumcocoylglutamat | 2% |
| PEG-40 hydriertes Rizinusöl | 0,5% |
| PEG-100 hydriertes Glycerylpalmitat | 1,5% |
| Natriumbenzoat | 0,45% |
| Natriumsalicylat | 0,2% |
| Citronensäure | 0,5% |
| Parfum | q.s. |
| Wasser | ad 100 |

Zusätzlich noch folgende weitere Rohstoffe: Perlglanz Wachse (1 % bis 3%), Trübungsmittel (Wachse oder Polymere) (0,5% bis 3%), Polyquaternium-10 (0,1% bis 0,3%), Trisodium EDTA (0,2% bis 0,6%)

In dieser Zubereitung wurde die Hautverträglichkeit der Rezeptur unter Beispiel 1, durch die Verwendung milderer Tenside (z.B. Natrium Myrethsulfat) und die Reduzierung der Gesamttensidkonzentration noch einmal verbessert.

### Beispiel 3:

| | |
|---|---|
| Natrium Laurethsulfat | 11% |
| Cocoamidopropylbetain | 4,3% |
| Natrium Lauroyl Sarcosinate | 2% |
| PEG-40 hydriertes Rizinusöl | 0,5% |
| PEG-100 hydriertes Glycerylpalmitat | 1,5% |
| Natriumbenzoat | 0,45% |
| Natriumsalicylat | 0,2% |
| Citronensäure | 0,5% |
| PEG-4 Rapeseedamide | 4% |
| PEG-9 Cocoglycerides | 1,6% |
| Hydroxypropyl Guarhydroxypropyltrimonium Chloride | 0,3% |
| Parfum | q.s. |
| Wasser | ad 100 |

Durch den Einsatz von Hautpflegestoffen und Moisturzern (z.B. PEG-4 Rapseedamide und PEG-9 Cocoglyceride) ist dieser Zubereitung speziell für den Einsatz einer Massagedusche geeignet.
Durch die Massage mit dem erfindungsgemäßen Duschprodukt können diese Hautpflegestoffe ihre Wirkung optimal entfalten. Ihre Wirkung kann zusätzlich erheblich gesteigert werden.

### Beispiel 4:

| | |
|---|---|
| Decylglucosid | 11% |
| Carbopol 1382 | 1,2% |
| Natriumhydroxid | 0,5% |
| Butylenglykol | 9% |
| Propylenglycol | 18% |
| Na₃HEDTA | 0,5% |
| Natriumbenzoat | 0,3% |
| Natriumsalicylat | 0,2% |
| Parfum | q.s. |
| Wasser | ad 100 |

Durch den Einsatz nur nichtionischer Tenside ist die Formel besonders hautfreundlich. Zusätzlich verbessert der Einsatz von Gelbildnern die Gleitfähigkeit des Massagekopfes bei Anwendung dieses Produktes erheblich. Beide Effekte sind für die Anwendung als Massagedusche von Vorteil.

### Beispiel 5:

| | |
|---|---|
| Paraffinöl | 25% |
| Sojaöl | 25% |
| Natriumlaurylethersulfat | 9% |
| Natriumbenzoat | 0,3% |
| Natriumsalicylat | 0,2% |
| Acrylates/C10-C30 Alkyl Acrylate Crosspolymer | 2% |
| Natriumhydroxid | 0,2% |
| Phenoxyethanol | 0,3% |
| Parabene | 0,1% |
| Parfum | q.s. |
| Wasser | ad 100 |

Der Einsatz von Gelbildnern verbessert die Gleitfähigkeit des Massagekopfes bei der Anwendung dieses Produktes erheblich. Der hohe Anteil an Hautpflegeölen ermöglicht schon eine aktive Rückfettung während der Massage. Durch diese Applikationsform werden die Hautpflegeöle in die oberen Hautschichten einmassiert, wodurch ihre Wirksamkeit verbessert wird.

### Beispiel 6:

| | |
|---|---|
| Sojaöl | 40% |
| Rizinusöl | 15% |
| Sonnenblumenöl | - |
| Weizemkeimöl | - |
| Zetesol 100 | 40% |
| Poloxamer 101 | 2% |
| Parfüm, Antioxidantien, Konservierungsstoffe | q.s. |
| Wasser | ad 100 |

Durch den erheblichen Ölanteil in dieser Zubereitung im Vergleich zu Beispiel 5, kommt es während der Anwendung zu einer verstärkten Rückfettung bei der Anwendung. Dadurch wird massagebedingten Irritationen vorgebeugt.

### Beispiel 7:

| | |
|---|---|
| Natrium Laurethsulfat | 11% |
| Natriumcocoamphoacetate | 4% |
| Polyethylene | 4% |
| Natriumcocoylglutamat | 1% |
| PEG-40 Glyceryl Cocoate | 0,5% |
| Phenoxyethanol+Methyldibromo Glutaronitrile | 0,2% |
| Hydroxypropyl Guarhydroxypropyltrimonium Chloride | 0,1% |
| PEG-3 Distearate | 2% |
| Magnesium Aluminium Silicate | 2,5% |
| Citronensäure | 0,5% |
| Parfum | q.s. |
| Wasser | ad 100 |

Durch den Einsatz von Peelingpartikeln kommt es bei dieser Zubereitung zu einer Verstärkung der Massagewirkung.

## Patentansprüche

1. Kosmetisches Reinigungsprodukt umfassend
- einen Massageapplikator für Flüssigkeiten, fließfähigen Formulierungen, Pasten und dergleichen, bestehend aus einem Behälterkörper (1) und einer Massagevorrichtung, welche wenigstens eine Massagenoppeneinrichtung (2) mit wenigstens einer ersten Oberfläche (2611) aufweist, die bei einer Massage die zu massierende Fläche kontaktiert, wobei zwischen dieser ersten Oberfläche (2611) der Massagenoppeneinrichtung (26) und dieser zu massierenden Fläche eine Kraft übertragbar ist und wobei im Bereich der ersten Oberfläche (2611) der Massagenoppeneinrichtung (2), die die zu massierende Fläche bei der Massage kontaktiert, eine vorbestimmte Härte und/oder Elastizität gegeben ist, **gekennzeichnet durch** wenigstens eine Einstelleinrichtung (26), mittels welcher wenigstens eine vorbestimmte Härte und/oder Elastizität einstellbar ist
- und eine kosmetischen Reinigungszubereitung.

2. Reinigungsprodukt nach Anspruch 1, **dadurch gekennzeichnet, dass** die Härte und/oder Elastizität der Massagenoppeneinrichtung (2) stufenlos einstellbar ist und die Härte- und/oder Elastizitätseinstellung durch verdrehen, horizontales oder vertikales verschieben eines innen liegenden Bauteils (25) erfolgt.

3. Reinigungsprodukt nach Anspruch 1, **dadurch gekennzeichnet, dass** die Geometrie im Bereich der ersten Oberfläche (2611) durch verdrehen, horizontales oder vertikales verschieben eines innen liegenden Bauteils (25) stufenlos einstellbar ist.

4. Reinigungsprodukt nach mindestens einem der vorangehenden Ansprüche, **dadurch gekennzeichnet, dass** dessen Massageapplikator wenigstens teilweise aus Kunststoff, wie Polyethylen oder Polypropylen, hergestellt ist.

5. Reinigungsprodukt nach mindestens einem der vorangehenden Ansprüche, **gekennzeichnet durch** eine Arretier-Einrichtung (2621), mittels welcher wenigstens ein erstes Element (26) gegenüber wenigstens einem zweiten Element (25) in einer vorbestimmten Relativposition arretierbar ist.

6. Reinigungsprodukt nach zumindest einem der vorangehenden Ansprüche, **dadurch gekennzeichnet, dass** mehrere Massagenoppen vorhanden sind, vorteilhaft in einem geometrischen Muster angeordnet sind, insbesondere konzentrisch angeordnet sind.

7. Reinigungsprodukt nach zumindest einem der vorangehenden Ansprüche, **dadurch gekennzeichnet, dass** die Massagenoppen oval sind.

8. Reinigungsprodukt nach zumindest einem der vorangehenden Ansprüche, **dadurch gekennzeichnet, dass** die Massagevorrichtung rund und abgewinkelt ist.

9. Reinigungsprodukt nach zumindest einem der vorgenannten Ansprüche, **dadurch gekennzeichnet**, das der Massagekopf sich zwecks Wiederverwendung auf neu gefüllte Behälter übertragen lässt.

10. Reinigungsprodukt nach mindestens einem der vorhergehenden Ansprüche, **dadurch gekennzeichnet**, das der Produktbehälter durch ein Ventil von der Massagevorrichtung getrennt ist.

11. Reinigungsprodukt nach mindestens einem der vorhergehenden Ansprüche, **dadurch gekennzeichnet, dass** die Viskosität und Gleitfähigkeit der kosmetische Reinigungszubereitung, insbesondere des Duschproduktes, auf der Haut auf die Massagevorrichtung abgestimmt ist.

12. Reinigungsprodukt nach zumindest einem der vorangehenden Ansprüche, **dadurch gekennzeichnet, dass** die Reinigungszubereitung in Form von Gelen, Emulsionen, Mikroemulsionen, Suspensionen, Dispersionen, Kolloiden, Pudern, Pulvern und/oder Pasten vorliegt.

13. Reinigungsprodukt nach zumindest einem der vorangehenden Ansprüche, **dadurch gekennzeichnet, dass** die Reinigungszubereitung anionische und/oder kationische und/oder nichtionische und/oder amphotere, beziehungsweise zwitterionischen Tenside enthält.

14. Reinigungsprodukt nach zumindest einem der vorangehenden Ansprüche, **dadurch gekennzeichnet, dass** die Reinigungszubereitung neben anionischen und/oder kationischen und/oder nichtionischen und/oder amphoteren, beziehungsweise zwitterionischen Tensiden auch anionische Cocoylglutamate enthält.

15. Reinigungsprodukt nach zumindest einem der vorangehenden Ansprüche, **dadurch gekennzeichnet, dass** die Reinigungszubereitung neben anionischen und/oder kationischen und/oder nichtionischen und/oder amphoteren, beziehungsweise zwitterionischen Tensiden auch ethoxylierte Fettsäurederivate (z. B. PEG-4 Rapeseedamide, PEG-9 Cocoglycerides) enthält.

16. Reinigungsprodukt nach zumindest einem der vorangehenden Ansprüche, **dadurch gekennzeichnet, dass** die Reinigungszubereitung neben anionischen und/oder kationischen und/oder nichtionischen und/oder amphoteren, beziehungsweise zwitterionischen Tensiden auch Triglyceride oder Mineralöle enthält.

17. Reinigungsprodukt nach zumindest einem der vorangehenden Ansprüche, **dadurch gekennzeichnet, dass** die Reinigungszubereitung neben anionischen und/oder kationischen und/oder nichtionischen und/oder amphoteren, beziehungsweise zwitterionischen Tensiden, zusätzlich Peelingkörper aus organischem oder synthetischen Materialien zur Abschilferung der oberen Schichten des Stratum Corneums enthält.

18. Verwendung eine Reinigungsproduktes nach mindestens einem der vorhergehenden Ansprüche als Duschprodukt.

## Claims

1. Cosmetic cleaning product comprising
- a massage applicator for liquids, fluid formulations, pastes and the like, comprising a container body (1) and a massage device which has at least one massage pin device (2) having at least one first surface (2611) which, on massaging, is in contact with the surface to be massaged, wherein a force can be transmitted between this first surface (2611) of the massage pin device (26) and this surface to be massaged, and wherein a predetermined hardness and/or elasticity exists in the region of the first surface (2611) of the massage pin device (2) which, on massaging, is in contact with the surface to be massaged, **characterized by** at least one adjusting device (26), by means of which at least one predetermined hardness and/or elasticity can be adjusted
- and a cosmetic cleaning formulation.

2. Cleaning product according to Claim 1, **characterized in that** the hardness and/or elasticity of the massage pin device (2) is infinitely adjustable and the hardness and/or elasticity is adjusted by turning, horizontal or vertical displacement of a component (25) on the inside.

3. Cleaning product according to Claim 1, **characterized in that** the elasticity and/or the geometry in the region of the first surface (2611), are infinitely adjustable by turning, horizontal or vertical displacement of a component (25) on the inside.

4. Cleaning product according to at least one of the preceding claims, **characterized in that** its massage applicator is produced at least partly from plastic, such as polyethylene or polypropylene.

5. Cleaning product according to at least one of the preceding claims, **characterized by** a stopping device (2621), by means of which at least one first element (26) can be stopped in a predetermined relative position with respect to at least one second element (25).

6. Cleaning product according to at least one of the preceding claims, **characterized in that** several massage pins are present and are advantageously arranged in a geometric pattern, in particular are arranged concentrically.

7. Cleaning product according to at least one of the preceding claims, **characterized in that** the massage pins are oval.

8. Cleaning product according to at least one of the preceding claims, **characterized in that** the massage device is round and angled.

9. Cleaning product according to at least one of the preceding claims, **characterized in that** the massage head can be transferred to newly filled containers for re-use.

10. Cleaning product according to at least one of the preceding claims, **characterized in that** the product container is separated from the massage device by a valve.

11. Cleaning product according to at least one of the preceding claims, **characterized in that** the viscosity and slip properties of the cosmetic cleaning formulation, in particular of the shower product, on the skin are matched to the massage device.

12. Cleaning product according to at least one of the preceding claims, **characterized in that** the cleaning formulation is in the form of gels, emulsions, microemulsions, suspensions, dispersions, colloids, dusting powder, powders and/or pastes.

13. Cleaning product according to at least one of the preceding claims, **characterized in that** the cleaning formulation comprises anionic and/or cationic and/or nonionic and/or amphoteric or zwitter-ionic surfactants.

14. Cleaning product according to at least one of the preceding claims, **characterized in that** the cleaning formulation also comprises anionic cocoylglutamate, in addition to anionic and/or cationic and/or nonionic and/or amphoteric or zwitter-ionic surfactants.

15. Cleaning product according to at least one of the preceding claims, **characterized in that** the cleaning formulation also comprises ethoxylated fatty acid derivatives (e.g. PEG-4 rape seed amide, PEG-9 cocoglycerides), in addition to anionic and/or cationic and/or nonionic and/or amphoteric or zwitter-ionic surfactants.

16. Cleaning product according to at least one of the preceding claims, **characterized in that** the cleaning formulation also comprises triglycerides or mineral oils, in addition to anionic and/or cationic and/or nonionic and/or amphoteric or zwitter-ionic surfactants.

17. Cleaning product according to at least one of the preceding claims, **characterized in that** the cleaning formulation additionally comprises peeling bodies of organic or synthetic materials for rubbing off the upper layers of the stratum corneum, in addition to anionic and/or cationic and/or nonionic and/or amphoteric or zwitter-ionic surfactants.

18. Use of a cleaning product according to at least one of the preceding claims as a shower product.

## Revendications

1. Produit nettoyant cosmétique comprenant
- un applicateur-masseur pour liquides, formulations fluides, pâtes et analogues, constitués d'un corps formant récipient (1) et d'un dispositif de massage, qui comprend au moins un dispositif (2) à doigts de massage comportant au moins une première surface (2611) qui, lors d'un massage, entre en contact avec la surface à masser, une force pouvant être transmise entre cette première surface (2611) du dispositif (26) à doigts de massage et cette surface à masser, une dureté et/ou une élasticité prédéterminées existant dans la zone de la première surface (2611) du dispositif (2) à doigts de massage, qui entrent en contact, lors du massage, avec la surface à masser, **caractérisé par** au moins un dispositif de réglage (26), à l'aide duquel il est possible de régler au moins une dureté et/ou une élasticité prédéterminées,
- et une préparation nettoyante cosmétique.

2. Produit nettoyant selon la revendication 1, **caractérisé en ce que** la dureté et/ou l'élasticité du dispositif (2) à doigts de massage peut être réglé d'une manière continue, et le réglage de la dureté et/ou de l'élasticité est réalisé par rotation, translation horizontale ou verticale, d'un composant intérieur (25).

3. Produit nettoyant selon la revendication 1, **caractérisé en ce que** la géométrie, dans la zone de la première surface (2611), peut être réglée d'une manière discontinue par rotation, translation horizontale ou verticale d'un composant intérieur (25).

4. Produit nettoyant selon au moins l'une des revendications précédentes, **caractérisé en ce que** son applicateur-masseur est fabriqué au moins partiellement en une matière plastique, telle que le polyéthylène ou le polypropylène.

5. Produit nettoyant selon au moins l'une des revendications précédentes, **caractérisé par** un dispositif de blocage (2621), à l'aide duquel il est possible de bloquer dans une position prédéfinie au moins un premier élément (26), par rapport à au moins un deuxième élément (25).

6. Produit nettoyant selon au moins l'une des revendications précédentes, **caractérisé en ce que** plusieurs doigts de massage sont présents, sont avantageusement disposés selon un motif géométrique, et en particulier ont une disposition concentrique.

7. Produit nettoyant selon au moins l'une des revendications précédentes, **caractérisé en ce que** les doigts de massage sont ovales.

8. Produit nettoyant selon au moins l'une des revendications précédentes, **caractérisé en ce que** le dispositif de massage est circulaire et replié.

9. Produit nettoyant selon au moins l'une des revendications précédentes, **caractérisé en ce que** la tête de massage peut, pour une réutilisation, être transférée sur des récipients rechargés.

10. Produit nettoyant selon au moins l'une des revendications précédentes, **caractérisé en ce que** le récipient à produit est séparé du dispositif de massage par une soupape.

11. Produit nettoyant selon au moins l'une des revendications précédentes, **caractérisé en ce que** la viscosité et le pouvoir glissant de la préparation nettoyante cosmétique, en particulier du produit pour la douche, sur la peau, sont adaptés au dispositif de massage.

12. Produit nettoyant selon au moins l'une des revendications précédentes, **caractérisé en ce que** la préparation nettoyante se présente sous forme de gels, d'émulsions, de micro-émulsions, de suspensions, de dispersions, de colloïdes, de poudres et/ou de pâtes.

13. Produit nettoyant selon au moins l'une des revendications précédentes, **caractérisé en ce que** la préparation nettoyante contient des tensioactifs anioniques et/ou cationiques et/ou non ioniques et/ou amphotères, ou zwittérioniques.

14. Produit nettoyant selon au moins l'une des revendications précédentes, **caractérisé en ce que** la préparation nettoyante contient aussi, outre des tensioactifs anioniques et/ou cationiques et/ou non ioniques et/ou amphotères, ou zwittérioniques, des cocoylglutamates anioniques.

15. Produit nettoyant selon au moins l'une des revendications précédentes, **caractérisé en ce que** la préparation nettoyante contient aussi, outre des tensioactifs anioniques et/ou cationiques et/ou non ioniques et/ou amphotères, ou zwittérioniques, des dérivés d'acides gras éthoxylés (par exemple un amide gras dérivé de l'huile de colza PEG-4, des coco-glycérides PEG-9).

16. Produit nettoyant selon au moins l'une des revendications précédentes, **caractérisé en ce que** la préparation nettoyante contient aussi, outre des tensioactifs anioniques et/ou cationiques et/ou non ioniques et/ou amphotères, ou zwittérioniques, des triglycérides ou des huiles minérales.

17. Produit nettoyant selon au moins l'une des revendications précédentes, **caractérisé en ce que** la préparation nettoyante contient, outre des tensioactifs anioniques et/ou cationiques et/ou non ioniques et/ou amphotères, ou zwittérioniques, des éléments exfoliants constitués de matériaux organiques ou synthétiques, pour assurer l'exfoliation des couches supérieures de la couche cornée.

18. Utilisation d'un produit nettoyant selon au moins l'une des revendications précédentes, en tant que produit pour la douche.
